# EUROPEAN PATENT SPECIFICATION

(11) **EP 1 607 048 B1**
(45) Date of publication and mention of the grant of the patent: **17.03.2010**
(21) Application number: 05253693.5
(22) Date of filing: 15.06.2005
(51) Int. Cl.: A61B 17/064, A61B 17/068

(54) **Improved surgical fastener**
Chirurgisches Befestigungselement
Dispositif de fixation chirurgicale

(30) Priority: 16.06.2004 US 870348
(43) Date of publication of application: 21.12.2005
(73) Proprietor: ETHICON ENDO-SURGERY, INC., Cincinnati, Ohio 45242 (US)
(72) Inventor: Huitema, Thomas W., Cincinnati Ohio 45241 (US); Stoeckel, Dieter, Los Altos California 94024 (US)
(74) Representative: Tunstall, Christopher Stephen

(56) References cited:
- FR-A- 2 797 275
- US-B1- 6 387 121
- US-B1- 6 638 297

## Description

### Field of the Invention

The present invention has application in conventional endoscopic and open surgical instrumentation as well application in robotic assisted-surgery. The present invention has even further relation to surgical clips and staples.

### Background of the Invention

In recent years surgery has markedly advanced through the performance of laparoscopic and endoscopic surgical procedures such as cholecystectomies, gastrostomies, appendectomies, and hernia repair. These procedures are accomplished through a trocar containing a sharpened obturator tip and a trocar hub or cannula. The trocar cannula is inserted into the skin to access the body cavity, by using the obturator tip to penetrate the skin. After penetration, the obturator is removed and the trocar cannula remains in the body. It is through this trocar cannula that surgical stapling and clipping instruments are placed. Once such trocar is Endopath® trocar manufactured by Ethicon Endo-Surgery, Inc. Cincinnati, Ohio.

The application of endoscopic surgical stapling and clipping instruments has been provided in such surgical procedures. One such endoscopic instrument, often referred to as an Endocutter, is capable severing tissue and providing homeostasis along both sides of the cut. An example of an Endocutter can be found in U.S. Patent 5,673,840 issued on October 7, 1997.

In the case of such Endocutter, the tissue is compressed between a lower jaw and an anvil. The lower jaw holds a cartridge that holds tiny drivers that house U-shaped staples. After the tissue is compressed, axial movement of the firing wedges forces the drivers and staples radially toward the anvil. This movement causes the staples to pierce the compressed tissue and strike curved pockets in the face and anvil. When the legs of the staples strike the anvil pockets, column buckling is initiated and they curl inward in a manner similar in concept to operation of a common office stapler. The anvil pocket geometry causes them to deform inward, forming a B-like shape as the legs of the staples are permanently deformed back on themselves. Often, two triple rows of staples are being simultaneously formed, with a knife following just behind the forming operation to separate the tissue between the two triple rows (lines) of staples. There are a number of problems associated with the forces required to deform the staples. The high forces require expensive materials and manufacturing techniques because the jaw and anvil structures need to be highly strong and rigid. In addition, the high forces require high firing wedge forces to deform the staples. The forces must be generated over the length of the staple line. The resulting total energy input limits the length of staple lines that can be formed by a human using a single hand squeezing motion. Many physicians find it difficult to fire an Endocutter.

Furthermore, it is difficult to use a single staple size that can provide homeostasis over a range of tissue thicknesses. With conventional metal staples, the staple legs tend to simply buckle part of the way back from the distal ends. This distal portion remains primarily straight. As a result, when the staples are deformed most extensively for very thin tissue, the straight portions of the staple legs pass beyond the flat base of the staple and the sharp points end up protruding out of the tissue where they can catch and lacerate tissue. If the tissue is very thick and only the distal portions of the staple legs are formed, the staple legs won't curve back on themselves and form the hook-like geometry required to hold the tissue in place.

One solution to above problems was to make a surgical fastener like that described in U.S. Patent 6,638,297, the preamble of claim 1 is based on this document. This document describes a surgical staple having first undeployed shape for loading into a stapler, and a second deployed shaped for connecting tissue together. The staple has a crown and first and second legs, one attached to each end of the crown. The legs extend from the crown in a direction substantially perpendicular to the longitudinal axis of the crown when the staple is in its first shape. The legs comprise first and second layers of material joined together. The first layer of material is a superelastic alloy having a relaxed configuration substantially in the staple's second shape. The second layer of material having a relaxed configuration substantially in the staple's first shape. The second layer of material has sufficient rigidity to keep the first layer in the first shape prior to the staple being deployed.

In the above mentioned reference an embodiment was described wherein titanium was used for the second linear elastic layer, and NITINOL used as the first layer or the core. Because the two materials are very close to each other electrochemically, the resulting wire forms are very corrosion-resistant. The difficulty is that the nickel, in the NITINOL, and the titanium form a number of intermetallic compounds and alloys with mixing ratios higher and lower than 50:50_{atomic}. Although there are a number of them, one in particular is the eutectic nickel-titanium alloy containing ~24% nickel and 76% titanium. This eutectic alloy is brittle and difficult to form. Those skilled in the arts will readily recognize that a certain amount of diffusion is bound to take place at the bonding interface between the NITINOL and titanium, especially if the two materials are heated to improve the bonding process. A bonding interface created between 50:50_{atomic} nickel-titanium NITINOL and nearly 100% titanium would be bound to create at least a very thin layer of the brittle 24:76 nickel-titanium eutectic material somewhere within the bond interface between the NITINOL and titanium. Other intermetallic compounds and alloys with more than 50%_{atomic} titanium could be expected to be created within the bonding zone as well. US 6,387,121 discloses a stent formed of three different layers.

### Summary of the Invention

In accordance with the present invention there is provided a medical fastener having first undeployed shape for loading into an applier, and a second deployed shape for connecting tissue together. The fastener includes a crown having two ends and first and second legs one attached to each end of the crown. The legs are separated from one another when the fastener is in the first shape. The legs comprise first, second and third layers of material joined together. The first layer of material is a superelastic alloy having a relaxed configuration substantially in the second shape of the fastener. The second layer of material comprising a linear elastic material having a relaxed configuration substantially in the first shape of the fastener. The second layer of material has sufficient rigidity to keep the first layer in the first shape prior to the fastener being deployed. The third layer of material is disposed between the first and second layers and is a material which substantially prevents diffusion between the first and second layers of material

### Brief Description of the Figures

The novel features of the invention are set forth with particularity in the appended claims. The invention itself, together with further objects and advantages thereof, may best be understood by reference to the following description, taken in conjunction with the accompanying drawings in which:
Figure 1 is a side view of a surgical stapler which can be used with the present invention.
Figure 2 is a plan view of a surgical staple made in accordance with the present invention and showing the staple in its undeployed shape.
Figure 3 is a cross-sectional view of the staple shown in Figure 2 taken along line 3-3.
Figure 4 is a plan view of the surgical staple shown in figure 1, but showing the staple in its deployed shape.

### Detailed Description of the Invention

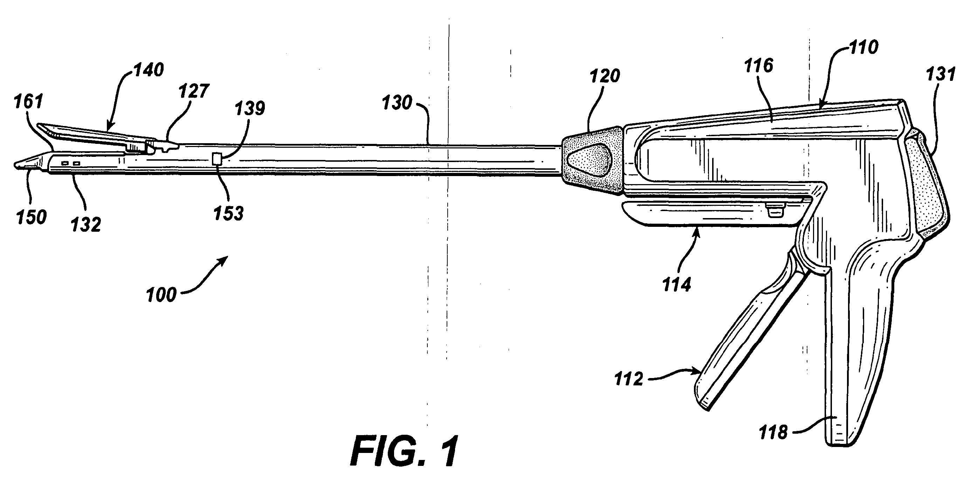

Referring now to the drawings wherein like numerals indicate the same elements throughout the views, there is shown in Figure 1 a surgical stapler, or Endocutter, 100 designed to be used with the present invention. Stapler 100 is of the kind described in the U.S. Patent 5,673,840. Stapler 100, which contains a handle portion 110, rotating means 120, a shaft portion 130, anvil portion 140, and cartridge assembly 150. A knife means (not shown) is slidable within the cartridge assembly 150 to cut tissue. In the handle portion 110 there is a first or closure trigger (also called a clamping trigger) 112, and second or firing trigger 114. The clamping trigger 112 causes the anvil portion 140 to come into proximity of the cartridge assembly 150. The firing trigger 114 causes staples to eject from the cartridge and form against the anvil portion 140. Trigger 114 also causes the knife means to move through the cartridge assembly 150, in order to cut tissue.

As will be appreciated by those skilled in the art, the below described surgical staple has equal application for use in open linear cutters, such as those described in U.S. Patent 4,520,817 issued to Green on June 4, 1985. In addition, as used herein staple refers to any type of substantially rigid and deformable surgical fasteners. Consequently, as will be appreciated by those skilled in the art, the below described staple has equal application for use in a clip applier or ligation device, such as the one described in U.S. Patent 5,447,513 issued to Davison et al. on September 5, 1995.

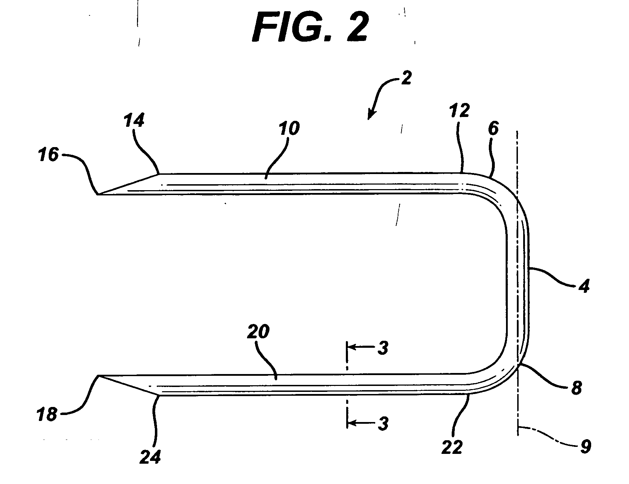

Referring now to Figure 2, there is shown a surgical fastener, which in this embodiment is shown as staple 2 made in accordance with the present invention, and designed to be loaded in a cartridge of the type described above as item 150. As will be discussed below, staple 2 has a first undeployed shape, and a second deployed shape. Figure 2 depicts staple 2 in its first undeployed shape. Staple 2 has a crown 4 having first and second ends 6 and 8 and a longitudinal axis 9 extending therebetween. Staple 2 also includes first and second legs 10 and 20. Legs 10 and 20 have first ends 12 and 22 which are attached to first and second ends 6 and 8 of crown 4. Legs 10 and 20 also have second ends 14 and 24 which extend from crown 4 in a direction generally perpendicular to longitudinal axis 9. Second ends 14 and 24 may include sharpened tips 16 and 18.

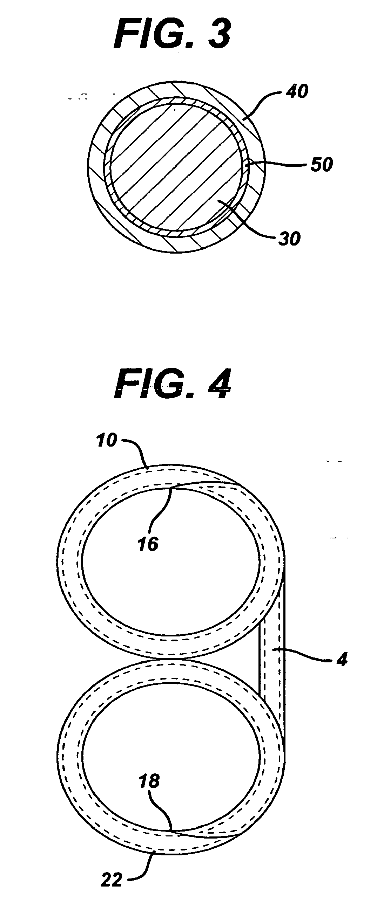

The material construction of staple 2 can best be described by referring to figure 3. As seen from the drawing, at least the legs, if not the entire staple, are formed from three coextensive layers of material 30, 40 and 50 joined together. As will be discussed in greater detail below, the first layer of material, or core, 30 is made from a superelastic alloy having a relaxed configuration substantially in the staple's second shape. The second layer of material 40, or shell, is made from a linear elastic material having a relaxed configuration substantially in the staples first shape . The second layer of material 40 has sufficient rigidity to keep the first layer in the first shape prior to the staple being deployed. The third layer of material 50 is preferably made from a material which substantially prevents diffusion between the first and second layers.

For purposes of this invention, the first and second layers of material are interchangeable. For example the first inner layer 30, or core, could be made from the linear elastic material, while the second outer layer 40, or shell is constructed from a superelastic material. Moreover, it is not necessary that the layers have circular cross-sections, but could take on any desired shape. In addition, it is not necessary that the cross section of the staple have the core/shell configuration. The layers could be juxtaposed and coextensive with each other, or have any other desired configuration.

The first layer 30 of material is preferably made from a superelastic or pseudoelastic alloy. One such type of material is commonly referred to as NITINOL. The nature of the superelastic transformations of shape memory alloys is discussed in "Engineering Aspects of Shape Memory Alloys", T W Duerig et al, on page 370, Butterworth-Heinemann (1990). A principal characteristic of shape memory alloys involves an initial increase in strain, approximately linearly with stress. This behavior is reversible, and corresponds to conventional elastic deformation. Subsequent increases in strain are accompanied by little or no increase in stress, over a limited range of strain to the end of the "loading plateau". The loading plateau stress is defined by the inflection point on the stress/strain graph. Subsequent increases in strain are accompanied by increases in stress. On unloading, there is a decline in stress with reducing strain to the start of the "unloading plateau" evidenced by the existence of an inflection point along which stress changes little with reducing strain. At the end of the unloading plateau, stress reduces with reducing strain. The unloading plateau stress is also defined by the inflection point on the stress/strain graph. Any residual strain after unloading to zero stress is the permanent set of the sample. Characteristics of this deformation, the loading plateau, the unloading plateau, the elastic modulus, the plateau length and the permanent set (defined with respect to a specific total deformation) are established, and are defined in, for example, "Engineering Aspects of Shape Memory Alloys", on page 376.

Non-linear superelastic properties can be introduced in a shape memory alloy by a process which involves cold working the alloy for example by a process that involves pressing, swaging or drawing. The superelastic properties are employed by the staple in its change of configuration between its first or undeployed/restrained shape, and its second or deployed/relaxed shape. An appropriate treatment can involve a combination of cold working (for example by swaging, drawing or, in particular by mandrel expansion) and heat treatment at a temperature that is less than the recrystallisation temperature of the alloy while the staple is constrained in the configuration resulting from the cold work. A plurality of the cold work and heat treatment steps can be used. The staple can then be deformed towards undeployed shape, the deformation being recoverable, substantially elastically. In this way, deformations of up to 8% strain can be imparted and recovered substantially elastically. The alloy for the first layer 30 is preferably manufactured such that it exhibits superelastic properties at body temperature.

Preferably NITINOL or Ni-Ti binary alloys for the first layer of material have a nickel content of at least about 50 atomic percent (hereinafter at. %), preferably at least about 50.5 at. %. The nickel content will usually be less than about 54 at. %, preferably less than about 52 at. %. As will be appreciated by those skilled in the art, the first layer can be made from other Ni-Ti based alloys, including alloys with ternary and quaternary additions. Examples of elements that can be incorporated in the alloy include Fe, Co, Cr, Al, Cu and V. Added elements can be present in amounts up to about 10 at. %, preferably up to about 5 at. %. Preferably the austenite finish temperature (Af) is below body temperature, and more preferably is around 0°C.

The second layer of material 40 is preferably made from a linear elastic material, such as iron, non-superelastic NITINOL, stainless steel or titanium. The second layer could also be made from a material which would impart radiopaque qualities to the staple so it could be seen better under x-ray. The yield strength of the second layer of material is set to be modestly higher than the recovery strength of the first layer of material.

The third layer of material 50 is preferably made from a material which substantially prevents diffusion between the first and second layers. The third layer 50 could be of an element which did not create brittle alloys or intermetallic compounds when blended with either the first or second material. Layer 50 preferably readily forms strong bonds with both first and second layers and is preferably electrochemically compatible with the first and second layers in the deployed shape so as not to induce electrochemical corrosion when implanted. In addition, third layer 50 is preferably biocompatible, both by itself and when bonded to the first and second layers.

When the first layer 30 is made from NITINOL and the second layer 40 is made from titanium, layer 50 can be made from materials such as tantalum and niobium. In manufacturing the wire, the NITINOL extrusion or wire drawing perform is preferably cleaned, then coated with a layer of 50. The barrier-coated NITINOL core would then be placed inside a close-fitting and very clean titanium cover piece. Well known procedures could be used to exclude oxygen and other embrittling gases and elements from the bonding surface and the materials would be bonded using a combination of thermal and mechanical means.

The thickness of layer 50 can be based on a number of considerations. The minimum thickness in the finished composite wire diameter could be based on the thinnest layer of material that was capable of reliably isolating the NITINOL from the titanium during both initial forming of the wire and subsequent thermal processing while fabricating the wire into components like staples or clips. The maximum barrier thickness could be based on the largest amount of the barrier element that could be present without measurably affecting the mechanical properties of the final fastener.

After a wire made of the three materials is formed, it can then be cut into a desired fastener size length segment. Thereafter the segment is cooled so that the NITINOL is substantially martensitic, and then the segment is deformed into its desired second/deployed shape, shown in Figure 4. The segment is then heat treated to shape set the NITINOL and partially stress relieve the Titanium. After the NITINOL in the wire has been shape-set, the staple can be straightened to the geometry depicted in figure 2 to form staple 2 which will then be loaded into and used in conventional surgical staplers.

The staple 2 combines shape-memory and linear-elastic materials such that the staple has some of the properties of shape-memory materials and some of the properties of linear-elastic materials. When deploying the staple, such as ejecting it from a cartridge onto an anvil, the sum of applied stresses and internally generated shape-memory recovery stresses exceed the yield strength of the linear-elastic material such that the staple will deform. When the loads are applied in such a fashion that they aid the shape-set material recovery stresses and the external load required to cause deformation will be lower than if the forces were applied to the linear-elastic portion of the staple alone.

As the staple is deployed, the staple would begin deforming and assuming the desired "B" shape at much lower loads than a conventional staple. This means that even at early stages of staple formation, the tips of the staple would take on a hook-like shape and eventually bend back upon themselves as shown in Figure 4. In contrast, a conventional staple would initially buckle closer to the middle of the staple and a major distal portion of the staple leg might stay straight and not even bend. As a result, when the staples are deformed most extensively for very thin tissue, the straight portions of the staple legs pass beyond the flat base of the staple and the sharp points end up protruding out of the tissue where they can catch and lacerate tissue. If the tissue is very thick and only the distal portions of the staple legs are formed, the staple legs won't curve back on themselves to form the hook-like geometry required to hold the tissue in place. The above mentioned staple and its associated geometry reduce these drawbacks.

Radial forming forces would remain lower for the above described staple throughout the forming process providing the staples were originally formed and shape-set at a formed height slightly less than the stapler could stroke to form them, even in thin tissue. This fundamental reduction in staple forming forces would have a ripple effect throughout the instrument because the tendency to force the anvil and cartridge channel apart would be reduced. Smaller, lighter components could be used for a given combination of staples lines and staple line length. In addition, it would make it feasible to design cantilevered-jaw staplers (the most common configuration) with longer staple line lengths than are currently feasible without making the components objectionably large and bulky.

The properties of the above mentioned staple could cause a manufacturer to increase the number of staples, and consequently the staple line length, that could be formed by a human using a single hand squeezing motion. This means the single-stroke, one-handed firing mechanism popular and economical on smaller staplers could be used on staplers with longer staple lines. This would provide a distinct advantage over the cost and complexity of staplers requiring multiple actuations to form all the staples or relying on powered designs that deprive the surgeon of tactile feedback during use.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. A medical fastener (2) having first undeployed shape for loading into an applier, and a second deployed shape for connecting tissue together, said fastener comprising:
a. a crown (4) having two ends (6,8), first and second legs (10,20) attached to each end of said crown, wherein said legs are separated from one another when said fastener is in said first shape; and
b. wherein said legs (10,20) comprise first and second layers (30,40) of material joined together, said first layer (30) of material comprising a superelastic alloy having a relaxed configuration substantially in said second shape, said second layer (40) of material comprising a linear elastic material having a relaxed configuration substantially in said first shape and having sufficient rigidity to keep said first layer (30) in said first shape prior to said fastener being deployed, **characterised in that:** said legs (10,20) further comprise third layers (50) of material, wherein said third layer (50) of material is disposed between said first and second layers (30,40) and comprises a material which substantially prevents diffusion between said first and second layers (30,40) of material.

2. The fastener of claim 1 wherein said second layer (40) comprises a material selected from at least one of the following materials: iron, non-superelastic nickel titanium alloy, stainless steel, titanium.

3. The fastener of claim 1 or 2 wherein said third layer (50) comprises a material selected from at least one of the following materials: tantalum and niobium

4. The fastener of any preceding claim wherein said first layer (30) comprises a nickel titanium alloy.

5. The fastener of any preceding claim wherein said first layer (30) has an Af temperature below 37°C.

6. The fastener of any preceding claim wherein said first layer (30) has an Af temperature below 0°C.

7. The fastener of any preceding claim wherein said second layer (40) of material is disposed concentrically around said first layer (30).

8. The fastener of any preceding claim wherein said legs (10,20) have a substantially circular cross-section.

9. The fastener of any preceding claim wherein said second shape is substantially a B-shape.

10. The fastener of any preceding claim wherein said fastener (2) is a staple.

## Patentansprüche

1. Medizinisches Befestigungsteil (2) mit einem ersten nicht-eingesetzten Zustand zum Laden in einen Applikator und einem zweiten eingesetzten Zustand zum Verbinden von Gewebe, wobei das Befestigungsteil umfasst:
a. einen Scheitel (4) mit zwei Enden (6, 8), einem ersten und einem zweiten Bein (10, 20), die an jedem Ende des Scheitels angebracht sind, wobei die Beine voneinander getrennt sind, wenn das Befestigungsteil in dem ersten Zustand ist; und
b. wobei die Beine (10, 20) eine erste und eine zweite Materialschicht (30, 40) umfassen, die aneinander gefügt sind, wobei die erste Materialschicht (30) eine superelastische Legierung mit einer entspannten Ausgestaltung im Wesentlichen in dem zweiten Zustand umfasst, und die zweite Materialschicht (40) ein linear elastisches Material mit einer entspannten Ausgestaltung im Wesentlichen in dem ersten Zustand und ausreichender Steifigkeit umfasst, um die erste Schicht (30) in dem ersten Zustand zu halten, bevor das Befestigungsteil eingesetzt wird, **dadurch gekennzeichnet, dass**:
die Beine (10, 20) eine dritte Materialschicht (50) umfassen, wobei die dritte Materialschicht (50) zwischen der ersten und zweiten Schicht (30, 40) angeordnet ist und ein Material umfasst, das im Wesentlichen eine Diffusion zwischen der ersten und der zweiten Materialschicht (30, 40) verhindert.

2. Befestigungsteil nach Anspruch 1, wobei die zweite Schicht (40) ein Material, aus wenigstens einem der folgenden Materialien umfasst: Eisen, nicht superelastische Nickeltitanlegierung, Edelstahl, Titan.

3. Befestigungsteil nach Anspruch 1 oder 2, wobei die dritte Schicht (50) ein Material, aus wenigstens einem der folgenden Materialien umfasst: Tantal und Niob.

4. Befestigungsteil nach einem der vorstehenden Ansprüche, wobei die erste Schicht (30) eine Nickeltitanlegierung umfasst.

5. Befestigungsteil nach einem der vorstehenden Ansprüche, wobei die erste Schicht (30) eine Af-Temperatur kleiner 37° C hat.

6. Befestigungsteil nach einem der vorstehenden Ansprüche, wobei die erste Schicht eine Af-Temperatur kleiner 0° C hat.

7. Befestigungsteil nach einem der vorstehenden Ansprüche, wobei die zweite Materialschicht (40) konzentrisch um die erste Schicht (30) angeordnet ist.

8. Befestigungsteil nach einem der vorstehenden Ansprüche, wobei die Beine (10, 20) einen im Wesentlichen kreisförmigen Querschnitt haben.

9. Befestigungsteil nach einem der vorstehenden Ansprüche, wobei der zweite Zustand im Wesentlichen eine B-Form ist.

10. Befestigungsteil nach einem der vorstehenden Ansprüche, wobei das Befestigungsteil (2) eine Klammer ist.

## Revendications

1. Dispositif de fixation médical (2) ayant une première forme non déployée lui permettant d'être chargé dans un applicateur, et une seconde forme déployée pour connecter ensemble des tissus, ledit dispositif de fixation comprenant :
a. une couronne (4) dotée de deux extrémités (6, 8), une première et une seconde pattes (10, 20) liées à chaque extrémité de ladite couronne, dans laquelle lesdites pattes sont séparées l'une de l'autre lorsque ledit dispositif de fixation se trouve dans la première forme ; et
b. dans lequel lesdites pattes (10, 20) comprennent une première et une deuxième couches (30, 40) de matériau assemblées, ladite première couche (30) de matériau comprenant un alliage super-élastique ayant une configuration détendue sensiblement dans ladite seconde forme, ladite deuxième couche (40) de matériau comprenant un matériau linéaire élastique ayant une configuration détendue sensiblement dans ladite première forme et ayant une rigidité suffisante pour conserver ladite première couche (30) dans ladite première forme avant que ledit dispositif de fixation ne soit déployé, **caractérisé en ce que** lesdites pattes (10,20) comprennent en outre une troisième couche (50) de matériau, dans lequel ladite troisième couche (50) de matériau est disposée entre lesdites première et deuxième couches (30, 40) et comprend un matériau qui empêche sensiblement la diffusion entre lesdites première et deuxième couches de matériaux.

2. Dispositif de fixation selon la revendication 1, dans lequel ladite deuxième couche (40) comprend un matériau choisi parmi au moins un des matériaux suivants : fer, alliage nickel titane non super-élastique, acier inoxydable, titane.

3. Dispositif de fixation selon la revendication 1 ou 2, dans lequel ladite troisième couche (50) comprend un matériau choisi parmi au moins un des matériaux suivants : tantale et niobium.

4. Dispositif de fixation selon l'une quelconque des revendications précédentes, dans lequel ladite première couche (30) comprend un alliage nickel titane.

5. Dispositif de fixation selon l'une quelconque des revendications précédentes, dans lequel ladite première couche (30) présente une température Af inférieure à 37°C.

6. Dispositif de fixation selon l'une quelconque des revendications précédentes, dans lequel ladite première couche (30) présente une température Af inférieure à 0°C

7. Dispositif de fixation selon l'une quelconque des revendications précédentes, dans lequel ladite deuxième couche (40) de matériau est disposée de manière concentrique autour de ladite première couche (30).

8. Dispositif de fixation selon l'une quelconque des revendications précédentes, dans lequel lesdites pattes (10, 20) ont une section sensiblement circulaire.

9. Dispositif de fixation selon l'une quelconque des revendications précédentes, dans lequel ladite deuxième forme est sensiblement une forme en B.

10. Dispositif de fixation selon l'une quelconque des revendications précédentes, dans lequel ledit dispositif de fixation (2) est une agrafe.
